# EUROPEAN PATENT SPECIFICATION

(11) **EP 3 338 769 B1**
(45) Date of publication and mention of the grant of the patent: **02.03.2022**
(21) Application number: 17207605.1
(22) Date of filing: 15.12.2017
(51) Int. Cl.: A61K 31/12, A61K 31/366, A61P 15/00, A61K 38/48, A61K 31/198, A61K 31/4525

(54) **FORMULATIONS FOR USE IN THE TREATMENT OF ENDOMETRIOSIS AND DISORDERS THEREWITH ASSOCIATED**
ZUSAMMENSETZUNGEN ZUR BEHANDLUNG DER ENDOMETRIOSE UND VERBUNDENEN STÖRUNGEN
FORMULATIONS UTILISÉES DANS LE TRAITEMENT DE L'ENDOMÉTRIOSE ET DES TROUBLES ASSOCIÉS

(30) Priority: 20.12.2016 IT 201600128994
(43) Date of publication of application: 27.06.2018
(73) Proprietor: Inpha Research S.r.l., 20122 Milano MI (IT)
(72) Inventor: Samaritani, Giuseppe, 23900 Lecco LC (IT); Castelli, Simone, 23900 Lecco LC (IT)
(74) Representative: Perani & Partners S.p.A.

(56) References cited:
- EP-A1- 2 789 345
- WO-A1-96/25939
- WO-A1-02/062367
- WO-A1-2014/111268
- US-A1- 2008 213 246
- US-A1- 2013 064 803

## Description

### Field of the invention

The present invention relates to an association of quercetin, curcumin and N-acetylcysteine, for use in the treatment of endometriosis and disorders associated therewith and relative formulations containing the above active ingredients in combination with suitable excipients and/or diluents.

### Background art

Endometriosis is a chronic degenerative medical condition, characterized by the presence of ectopic endometrium and its stromal component outside the uterine cavity and most frequently just before ovaries and pelvis.

This disease can affect women even at a very early age and theoretically even before or at the same time of menarche and continue until the menopause.

The severity of symptoms such as pain and dysmenorrhea can be very variable and this determines a diagnostic assessment of the disease that is not always early, arriving in many cases only as a result of the persistence of a state of non-pregnancy that is a prelude to gynecological analyses.

The disease is often cause of severe hypofertility of the woman or even complete sterility and for this reason, it is considered a disease of great social and emotional impact.

From an epidemiological point of view, it is estimated that the number of European women suffering from endometriosis is close to 10% in those of reproductive age and in Italy it is estimated that at least 3 million women are affected. Approximately 30% to 40% of women with endometriosis are sterile and therefore this disease is considered one of the first and most serious causes of female infertility.

From a pathological point of view, the formation of ectopic tissue of an endometrical nature in districts such as ovaries, e pelvis and intestine can cause severe bleeding even during the menstruation, with the same hormone-dependent mechanism as that occurring when physiological menstrual bleeding takes place in the course of exfoliation of the uterine decidua. Precisely as a consequence of this physiopathological mechanism, the haemorrhage determines the release of irritating and pro-inflammatory substances that produce local irritation, with pain and spasms of the smooth muscles. The continuous irritation of the muco-epithelial tissues as a consequence produces anatomical and pathological adherences and abnormalities in the tissues involved. Therefore, metrorrhagia and menorrhagia can also occur, even in a severe degree. In many cases, over 60%, pain and discomfort reported by the patients is of a high degree and is often incapacitating, with evident repercussions on the quality of life and with repercussions on work productivity.

The diagnosis is clinical, supported by ultrasound and magnetic resonance imaging. Pharmacological therapy, alongside analgesic and anti-inflammatory drugs (NSAIDs) to manage pain, involves the use of drugs adapted to reduce or eliminate the estrogenic activity (GnRH antagonists) or of drugs with androgenic activity, such as danazol. In the first case, a sort of chemical menopause is induced, with obvious repercussions on the reproductive sphere of the woman of fertile age, in the the second case the risk is verified of serious and severe side effects including acne, hirsutism and increase of body weight. Alternatively, there is surgical therapy that involves laparotomy or laparoscopy with the aim of surgically removing the ectopic tissue sites.

Alongside basic medical and pharmacological therapy, there are nutraceutical approaches that have shown efficacy in animals and humans in reducing the inflammatory component (cytokines release) and inhibiting the proliferation of ectopic tissue. In particular, substances such as Curcumin have demonstrated, in human endometrium models, a significant anti-inflammatory activity especially as regards the inhibition of the release of adhesion molecules and TNF-α [1], a cytokine that plays a key role in chronic inflammation linked to endometriosis. Curcumin is also able to reduce the growth and proliferation of endometrial epithelial cells through a mechanism to inhibit the release of estradiol [2]. Furthermore, Curcumin exerts an inhibitory action of the proliferation of the endometrium through a mechanism of inhibition of the release of MMP-3 and of pro-apoptosis p53dependent and inhibition of the release of VEGF with consequent reduction of tissue vascularization [3,4]. In this context, also the use of N-Acetylcysteine (NAC) is quite promising, giving evidence in women suffering from endometriosis, of a reduction in the volume of cysts at the end of treatment that allowed excluding laparoscopic surgery in a good number of treated subjects as well as a significant increase in pregnancies in the treated group compared to the control group [5]. Finally, of certain clinical-applicative interest, the use of Quercetin, which in studies conducted on animal model has been shown to significantly reduce the expression of estrogen (Erα, ERβ) and progesterone (PR) receptors in the hypothalamus and in the endometrium, thus reducing the bond of the same hormones in tissue and consequently the volume of the ectopic tissue [6].

WO2014111268 discloses a pharmaceutical or nutritional association comprising curcumin and quercetin with other active components for use in prevention or treatment of bladder, pelvic and urogenital apparatus pathologies. Endometriosis is therein mentioned in a long list of possible diseases, but the experimental data in such patent only relate to the treatment of bladder neoplasm.

EP2789345 illustrates a composition containing N-acetyl cysteine, alpha-lipoic acid, and bromelain.

### Summary of the invention

The present invention relates to an association of substances having an anti-inflammatory, anti-proliferative and anti-angiogenetic activity of plant and amino acid origin, capable of significantly reducing the inflammation and hyperproliferation of the endometrial ectopic tissue during endometriosis, preferably associated with substances capable of improving the oral bioavailability thereof.

The object of the present invention is therefore:
a) an association consisting of Curcumin, Quercetin and N-acetylcysteine for use in the adjuvant treatment of endometriosis. A further object of the present invention is
b) oral formulations whereby the aforesaid association is administered in combination with suitable excipients and/or diluents.

In particular, when the above association is administered in such oral formulations in combination with at least one enteric absorption promoter selected from Bromelain, at least one Sucrester, L-Arginine base and Piperine and even better if they contain all four of the above promoters, it demonstrates significant anti-inflammatory and cyst volume reduction efficacy associated with endometriosis at dosages of the individual active ingredients much lower than those described in literature. This efficacy is due to a mechanism of promoting enteric absorption not previously described, original and not explainable as the sum of the action of the individual promoters.

### Detailed description of the invention

For the purposes of the present invention, the verbs "comprise" and "contain" used several times in the description and in the claims both for invention a) and b), as set forth in the summary, have the meaning that said association of invention a) and/or said formulation b), in addition to the components explicitly listed, may include further components.

For the purposes of the present invention, the definition "consisting of", which will be found in the remainder of the present description, in particular with reference to invention a), indicates that the association contemplated in invention a) does not include further components besides those shown.

The association of the substances Curcumin, Quercetin and of N-acetylcysteine guarantees a solid rationale in the reduction of the inflammatory-congestive and hyperproliferative component of the endometrium and to desensitize it to the action of estrogens and progesterone.

Therefore, the association for use in the treatment of endometriosis according to the present invention preferably consists of Curcumin and Quercetin, or alternatively it preferably consists of Curcumin, Quercetin and N-acetylcysteine.

The present association, although effective, especially if administered orally would require rather high dosages to achieve clinically optimal effects, dosages that sometimes do not allow the necessary compliance of intake for the patient and that can cause unwelcome side effects, especially on patients who show sensitivity against one or more of the active ingredients present in the association, which have altered hepatic or renal function or that follow drug therapy.

As reported above, the Applicant has found that it is possible to overcome the aforesaid drawbacks with the above association administered in an oral formulation, in combination with suitable excipients and/or diluents, the excipients whereof contain at least one absorption promoter selected from Bromelain, at least one Sucrestere, L-Arginine base and Piperine. According to a preferred embodiment of the invention, such association is administered in oral formulations in combination with suitable excipients, wherein the suitable excipients are all four absorption promoters - Bromelain, a Sucrester, L-arginine base and Piperine.

According to a further preferred embodiment, the oral formulations, besides containing at least one of the above enteric absorption promoters, are also gastroresistant and in particular are in the form of a tablet coated with a gastroresistant polymeric film or in the form of a hard or soft capsule (soft gel), the shell whereof consists of conventional gastroresistant polymers. This type of formulation is therefore capable of releasing said active ingredients in the small intestine tract in a highly bioavailable form, due to the presence of at least one of the above enteric absorption promoters, even more preferably due to the presence of all four promoters.

In particular, the one or more enteric absorption promoters are contained in each of said oral formulations- in the following quantities:
- Bromelain titre > 2000 GDU from a minimum of 10 to a maximum of 50 mg per dose of intake;
- Piperine from Black Pepper from a minimum of 0.1 to a maximum of 5.0 mg per dose of intake;
- L-Arginine base, from a minimum of 5 to a maximum of 50 mg per dose of intake;
- at least one Sucrester, where by sucrester it is meant at least one sucrose ester with fatty acids, belonging to the category of food excipients designated by the initials E473, from a minimum of 5 to a maximum of 50 mg per dose of intake.

The above absorption promoters, preferably in association with each other, have surprisingly shown to evoke the beneficial anti-inflammatory and anti-proliferative effects on patients affected by the clinical conditions described above (with specific reference to endometriosis), reducing significantly, in the first place pain associated with dysmenorrhea and bleeding volume in the menstrual phase, at doses of the active ingredients significantly lower than those mentioned in literature, in the absence of the above promoters. In particular, it has been seen that the combination of Bromelain, Piperine, L-Arginine base and at least one Sucrester, at the dosages shown in the following formulation example, allows reducing the dosages of Curcumin from 500 to 200 mg and Quercetin from 500 to 250 mg.

The permanence of the efficacy against a significant reduction in the dosages of the active ingredients can be explained by the absorpion promoting effect exerted by the association of Bromelain, black pepper, at least one Sucrester, L-Arginine base that produce a synergistic modulatory effect on the main anatomo-physiological mechanisms that intervene, hindering the enteric absorption of the active ingredients.

In particular, Bromelain has been shown to improve the oral bioavailability of low molecular weight heparins, increasing their penetration through the intestinal epithelium [7] and, probably, this effect can be explained in light of its proteolytic activity on the proteins that make up the enteric mucus (mucins) [8]. L-Arginine has been shown to promote the entry into the circulation of molecules with high molecular weight, modifying the structure of actin filaments at the level of tight junctions closed among e enterocytes (zonula occludens) [9,10].

Sucresters act by modulating the activity of some trans-membrane transporters and altering the permeability of the cell membrane [11].

Piperine acts by modulating the activity of hepatic and intestinal cytochromes, thus reducing the formation of less active metabolites and promoting their transport in the circulation (this mechanism is particularly important in the case of Curcumin [12]). Increasing the bioavailability of the active ingredients mentioned in association with the above enteric absorption promoters should not be considered obvious or easily inferred by a man skilled in the art, nor intended as a mere additive sum of their activities, since such promoters act on very different mechanisms and scientific literature has individually established the effectiveness thereof on substrates substantially different from those of the present invention. Therefore, the synergy of action in terms of improving bioavailability must be reasonably linked to synergistic mechanisms currently not yet easily described or hypothesised.

For example, L-Arginine, described in literature as being able to transiently alter the integrity of cell cytoskeletal structures such as the actin filaments of the zonula occludens of the enterocyte, could also create the alkaline pH conditions that make the polyphenol substances such as Curcumin, notoriously insoluble or poorly soluble in water, much more soluble in enteric fluids and, for this reason, more diffusible through the paracellular pathway. Furthermore, the sucrester, known as an enteric absorption promoter due to the increased diffusibility of the enterocyte cell membrane, could also create the conditions for dispersing Quercetin and Curcumin itself in micellar structures able to make them more absorbable within the enterocyte. The final overall effect of said promoters therefore, due to the final effectiveness of the composition described above, must be considered unforeseen, not previously described and original.

This unexpected and surprising increase in the efficacy of the formulation on the inflammatory-congestive and hyper-proliferative conditions of endometriosis allows treating patients who cannot take anti-inflammatory non-steroidal or steroidal drugs (renal, hypertensive or hepatic complications, gastric intolerance and osteoporosis) or anti-estrogens or androgens (cardio-vascular, dysmetabolic and oncological contraindications), with an effective preparation, of good tolerability and substantially free from significant side effects, such as to make it suitable also for patients who have already undergone laparoscopic or laparotomic ablative surgical treatments, as an adjuvant treatment.

The oral formulations according to the present invention can be food or dietetic supplements or actual pharmaceutical formulations.

According to a particularly preferred embodiment, in the formulations suitable for oral administration according to the present invention the association consists of:
- from 100 to 300 mg, more preferably 200 mg of Curcumin, for example from Curcuma Longa (minimum titre in Curcuminoids 90%);
- from 100 to 300 mg, and more preferably 250 mg of Quercetin also as the dihydrate form extracted for example from Sophora Japonica or from Dimorphandra Mollis and a minimum titre of 80%;
- 100 to 300 mg of N-Acetylcysteine and more preferably 150 mg of N-Acetylcysteine;
   and in combination with at least one of the following absorption promoters in the following preferred quantities:
- 10 to 50 mg and more preferably 30 mg of Bromelain with a minimum titre of 2000 GDU;
- 10 to 50 mg, more preferably 20 mg of L-Arginine base
- between 10 and 50 mg, more preferably 30 mg of a Sucrester, intended as sucrose ester with fatty acids and belonging to the group of food excipients E473;
- between 0.1 and 5.0 mg, more preferably 3.0 mg of piperine from vegetable sources, for example from black pepper (Piper Nigrum).

The above pharmaceutical formulations or food supplements are preferably made in the form of a filmed tablet with gastroresistant polymers or in the form of a gastroresistant hard or soft capsule (soft-gel) where conventional polymers are used to obtain gastroresistance, such as shellac, copolymer derivatives of methacrylic acid (Eudragit) and others suitable for this purpose.

The composition of a gastro-resistant oral formulation according to the present invention in the form of a filmed tablet with shellac is given for illustrative but nonlimiting purposes.

### Example 1

**Dimorphandra Mollis fruit**

| | |
|---|---|
| Quercetin dihydrate Tit. 95% | 263 mg (equivalent to 250 mg of Quercetin) |
| Curcuma Longa root curcumin Tit. 95% | 210 mg (equivalent to 200 mg of Curcumin) |
| Bromelain 2500 GDU | 30.0 mg |
| L-arginine base | 20.0 mg |
| Piper Nigrum Tit. 95% Piperine | 3.16 mg (equivalent to 3 mg Piperine) |
| Sucrester (one or more belonging to E473) | 30.0 mg |
| Excipients for gastro-resistance (SHELLAC) | as needed |
| Excipients for compression | as needed to 1250 mg |

### Example 2

**Dimorphandra Mollis fruit**

| | |
|---|---|
| Quercetin dihydrate Tit. 95% | 263 mg (equivalent to 250 mg of Quercetin) |
| Curcuma Longa root curcumin Tit. 95% | 210 mg (equivalent to 200 mg of Curcumin) |
| N-Acetylcysteine | 150 mg |
| Bromelain 2500 GDU | 30.0 mg |
| L-arginine base | 20.0 mg |
| Piper Nigrum Piperine Tit. 95% | 3.16 mg (equivalent to 3mg piperine) |
| Sucrester (one or more belonging to E473) | 30.0 mg |
| Excipients for gastro-resistance (SHELLAC) | as needed |
| Excipients for compression | as needed to 1250 mg |

### Bibliography

1. Kim KH. Curcumin attenuates TNF-α-induced expression of intercellular adhesion molecule-1, vascular cell adhesion molecule-1 and proinflammatory cytokines in human endometriotic stromal cells. Phytother Res. 2012 Jul;26(7):1037-47;
2. Zhang Y. Curcumin inhibits endometriosis endometrial cells by reducing estradiol production. Iran J Reprod Med. 2013 May;11(5):415-22;
3. Jana S. Curcumin as anti-endometriotic agent: implication of MMP-3 and intrinsic apoptotic pathway. Biochem Pharmacol. 2012 Mar 15;83(6):797-804;
4. Zhang Y. Inhibitory effect of curcumin on angiogenesis in ectopic endometrium of rats with experimental endometriosis. Int J Mol Med. 2011 Jan;27(1):87-94;
5. Zhang X. Inhibition effect and mechanisms of quercetin on surgically induced endometriosis. Sichuan Da Xue Xue Bao Yi Xue Ban. 2009 Mar;40(2):228-31, 244;
6. Yang Cao. Preliminary Study of Quercetin Affecting the Hypothalamic-Pituitary-Gonadal Axis on Rat Endometriosis Model. Evidence-Based Complementary and Alternative Medicine. Volume 2014, Article ID 781684, 12 pages;
7. Grabovac V Bernkop-Schnürch A. Improvement of the intestinal membrane permeability of low molecular weight heparin by complexation with stem bromelain. Int J Pharm. 2006 Dec 1;326(1-2):153-9. Epub 2006 Jul 4;
8. Amini A et al. Depletion of mucin in mucin-producing human gastrointestinal carcinoma: Results from in vitro and in vivo studies with bromelain and N-acetylcysteine. Oncotarget. 2015 Oct 20;6(32):33329-44;
9. Nusrat Abbas Motlekar, Kalkunte Srirangachar Srivenugopal, Mitchell S. Wachtel, and Bi-Botti Celestin Youan. Modulation of gastrointestinal permeability of low-molecularweight heparin by L-arginine: in-vivo and invitro evaluation. J Pharm Pharmacol. 2006 May; 58(5): 591-598;
10. Xue XY et al. Promoting effects of chemical permeation enhancers on insulin permeation across TR146 cell model of buccal epithelium in vitro. Drug Chem Toxicol. 2012 Apr;35(2):199-207;
11. Kiss L et al. Sucrose esters increase drug penetration, but do not inhibit p-glycoprotein in caco-2 intestinal epithelial cells. J Pharm Sci. 2014 Oct;103(10):3107-19;
12. Berginc K, Trontelj J, Basnet NS, Kristl A. Physiological barriers to the oral delivery of curcumin. Pharmazie. 2012 Jun;67(6):518-24;

## Claims

1. Association consisting of Curcumin, Quercetin and N-acetylcysteine having anti-inflammatory and antiproliferative activity for use in the treatment of endometriosis.

2. Association for the use according to claim 1, wherein said association is administered in oral formulations in combination with suitable excipients and/or diluents, - wherein the excipients comprise at least one enteric absorption promoter selected from: Bromelain, at least one Sucrester, L-Arginine base and Piperine.

3. Association for the use according to claim 2, wherein said suitable excipients comprise as enteric absorption promoters at the same time: Bromelain, a Sucrester, L-arginine base and Piperine.

4. Association for the use according to anyone of claims 1-3, wherein in the said oral formulation:
a) Quercetin is present in a variable dosage between 100 and 300 mg and more preferably 250 mg per dose and is in the form of plant extracts with titre not lower than 80% in Quercetin or Quercetin bihydrated;
b) Curcumin is present in a variable dosage between 100 and 300 mg and more preferably 200 mg per dose in the form of plant extracts with curcumin titre not lower than 90%, and
c) N-acetylcysteine is present in variable dosage between 100 and 300 mg and more preferably 150 mg.

5. Association for the use according to any one of claims 3 and 4, wherein:
i) Bromelain has a titre of at least 2000 GDU and is present in a variable dosage between 10 and 50 mg and more preferably 30 mg per dose;
ii) L-Arginine base has a titre of at least 2000 GDU and is present in a variable dosage between 5 and 50 mg and more preferably 20 mg per dose;
iii) at least one Sucrestere and is present in a variable dosage between 5 and 50 mg and more preferably 30 mg per dose;
iv) Piperine is present in a variable dosage between 0.1 and 5.0 mg and more preferably 3.0 mg per dose in the form of plant extracts.

6. Association for the use according to anyone of claims 1-5, wherein said oral formulation is in the form of tablets coated with gastro-resistant polymeric film or rigid or soft capsules whose shell contains a gastro-resistant material.

7. Association for the use according to anyone of claims 1-6 wherein said oral formulations are in the form of: food or dietary supplement or pharmaceutical formulation.

## Patentansprüche

1. Assoziation bestehend aus Curcumin, Quercetin und N-Acetylcystein mit entzündungshemmender und antiproliferativer Aktivität für die Verwendung zur Behandlung von Endometriose.

2. Assoziation zur Verwendung nach Anspruch 1, wobei die Assoziation in oralen Formulierungen in Kombination mit geeigneten Hilfsstoffen und/oder Verdünnungsmitteln verabreicht wird, - wobei die Hilfsstoffe mindestens einen magensaftresorptionsfördernden Stoff umfassen, ausgewählt aus: Bromelain, mindestens ein Sucrester-, L- Arginin-Base und Piperin.

3. Assoziation zur Verwendung nach Anspruch 2, wobei die geeigneten Hilfsstoffe gleichzeitig als magensaftresorptionsfördernde Mittel umfassen: Bromelain, ein Sucrester, L-Arginin-Base und Piperin.

4. Assoziation zur Verwendung nach einem der Ansprüche 1-3, wobei in der oralen Formulierung:
a) Quercetin in einer variablen Dosierung zwischen 100 und 300 mg und vorzugsweise 250 mg pro Dosis und in Form von Pflanzenextrakten mit einem Titer von mindestens 80 % an Quercetin oder Quercetin bihydriert vorliegt;
b) Curcumin in einer variablen Dosierung zwischen 100 und 300 mg und vorzugsweise 200 mg pro Dosis in Form von Pflanzenextrakten mit einem Curcumin-Titer von mindestens 90 % vorliegt, und
c) N-Acetylcystein in einer variablen Dosierung zwischen 100 und 300 mg, vorzugsweise 150 mg vorliegt.

5. Assoziation zur Verwendung nach einem der Ansprüche 3-4, wobei:
i) Bromelain einen Titer von mindestens 2000 GDU hat, und in einer variablen Dosierung zwischen 10 und 50 mg und vorzugsweise 30 mg pro Dosis vorliegt;
ii) L-Arginin-Base einen Titer von mindestens 2000 GDU aufweist und in einer variablen Dosierung zwischen 5 und 50 mg und vorzugsweise 20 mg pro Dosis vorliegt;
iii) mindestens ein Sucrester und in einer variablen Dosierung zwischen 5 und 50 mg und vorzugsweise 30 mg pro Dosis vorliegt;
iv) Piperin in einer variablen Dosierung zwischen 0,1 und 5,0 mg und vorzugsweise 3,0 mg pro Dosis in Form von Pflanzenextrakten vorliegt.

6. Assoziation zur Verwendung nach einem der Ansprüche 1 bis 5, wobei die orale Formulierung in Form von Tabletten, die mit einem gastro-resistenten Polymerfilm überzogen sind, oder von Hart- oder Weichkapseln, die in ihrer Hülle ein gastroresistentes Material enthalten, vorliegt.

7. Assoziation zur Verwendung nach einem der Ansprüche 1-6, wobei die oralen Formulierungen in Form von: Nahrungsmitteln oder Nahrungsergänzungsmitteln oder pharmazeutischen Formulierungen vorliegen.

## Revendications

1. Association constituée de curcumine, de quercétine et de N-acétylcystéine ayant une activité anti-inflammatoire et antiproliférative destinée à l'utilisation dans le traitement de l'endométriose.

2. Association destinée à l'utilisation selon la revendication 1, dans laquelle ladite association est administrée dans des formulations orales en combinaison avec des excipients et/ou des diluants appropriés, dans lesquelles les excipients comprennent au moins un promoteur d'absorption entérique choisi parmi : la bromélaïne, au moins un sucroester, la L-arginine base et la pipérine.

3. Association destinée à l'utilisation selon la revendication 2, dans laquelle lesdits excipients appropriés comprennent en tant que promoteurs d'absorption entérique en même temps : bromélaïne, un sucroester, L-arginine base et pipérine.

4. Association destinée à l'utilisation selon l'une quelconque des revendications 1 à 3, dans laquelle dans ladite formulation orale :
a) la quercétine est présente à une dose variable comprise entre 100 et 300 mg et plus préférablement 250 mg par dose et se présente sous la forme d'extraits végétaux titrant au moins 80% en quercétine ou en quercétine bihydratée ;
b) la curcumine est présente à une dose variable comprise entre 100 et 300 mg et plus préférablement 200 mg par dose sous forme d'extraits végétaux titrés à pas moins de 90% en curcumine, et
c) la N-acétylcystéine est présente à doses variables comprises entre 100 et 300 mg et plus préférentiellement 150 mg.

5. Association destinée à l'utilisation selon l'une quelconque des revendications 3 et 4, dans laquelle :
i) la bromélaïne est titrée à au moins 2000 GDU et est présente à une dose variable comprise entre 10 et 50 mg et plus préférablement 30 mg par dose ;
ii) la L-arginine base a un titre d'au moins 2000 GDU et est présente à une dose variable comprise entre 5 et 50 mg et plus préférablement 20 mg par dose ;
iii) au moins un sucroester et est présent à une dose variable comprise entre 5 et 50 mg et plus préférablement 30 mg par dose ;
iv) la pipérine est présente à une dose variable comprise entre 0,1 et 5,0 mg et plus préférablement 3,0 mg par dose sous forme d'extraits végétaux.

6. Association destinée à l'utilisation selon l'une quelconque des revendications 1 à 5, dans laquelle ladite formulation orale est sous la forme de comprimés enrobés d'un film polymère gastro-résistant ou de capsules rigides ou molles dont l'enveloppe contient un matériau gastro-résistant.

7. Association destinée à l'utilisation selon l'une quelconque des revendications 1 à 6, dans laquelle lesdites formulations orales sont sous la forme de : un complément alimentaire ou diététique ou une formulation pharmaceutique.
